(19) Europäisches Patentamt
European Patent Office
Office européen des brevets

(11) EP 3 311 790 B1

(12) **EUROPEAN PATENT SPECIFICATION**

(45) Date of publication and mention of the grant of the patent:
**06.11.2019 Bulletin 2019/45**

(21) Application number: **16814078.8**

(22) Date of filing: **24.05.2016**

(51) Int Cl.:
***A61K 8/34*** *(2006.01)* ***A61K 8/362*** *(2006.01)*
***A61K 8/39*** *(2006.01)* ***A61K 8/46*** *(2006.01)*
***A61K 8/73*** *(2006.01)* ***A61Q 5/00*** *(2006.01)*
***A61Q 5/02*** *(2006.01)* ***A61Q 5/12*** *(2006.01)*

(86) International application number:
**PCT/JP2016/065259**

(87) International publication number:
**WO 2016/208307 (29.12.2016 Gazette 2016/52)**

(54) **HAIR TREATMENT AGENT**

HAARBEHANDLUNGSMITTEL

AGENT DE TRAITEMENT CAPILLAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.06.2015 US 201562182793 P**
**29.09.2015 US 201562234144 P**

(43) Date of publication of application:
**25.04.2018 Bulletin 2018/17**

(73) Proprietor: **Kao Corporation**
**Chuo-ku**
**Tokyo 103-8210 (JP)**

(72) Inventors:
- **SCHROTT, Adam**
**Cincinnati, Ohio 45214 (US)**
- **SHIBUYA, Akiko**
**Tokyo 131-8501 (JP)**
- **AFWA, Firdausi**
**Tokyo 131-8501 (JP)**
- **HERLAMBANG, Stephanie**
**Tokyo 131-8501 (JP)**
- **NAGASE, Shinobu**
**Tokyo 131-8501 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 213 277**
**WO-A1-2010/016591**
**JP-A- 2004 256 551**
**JP-A- 2007 112 716**
**JP-A- 2007 161 605**
**JP-A- 2010 001 249**
**US-A1- 2005 191 263**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

[Field of the Invention]

The present invention relates to a hair treatment agent.

[Background of the Invention]

Hair is exposed on a routine basis to damage factors such as friction applied by e.g., washing and brushing and heat applied by e.g., a hair dryer and a hair iron. Recently, fashion-conscious peoples have commonly enjoyed changing appearance of hair, for example, by dying hair with various colors (e.g., coloring) and changing a hair style with help of a chemical treatment (e.g., permanent wave). Because of this, as these treatments are frequently applied, damages are further accumulated to hair. These hair damages cause hair troubles including hair-cuticle peeling, difficulty in styling and spreading of hair under high humidity.

To deal with these hair troubles, Patent Document 1 proposes a hair cosmetic composition, which contains a liquid crystalline structure formed of a cationic surfactant and a solid oily component; an organic acid; a protein; and a highly polymerized dimethylpolysiloxane, and reduces friction during hair washing, thereby reducing the amount of cuticle peeled off. Patent Document 2 proposes a hair cosmetic, which contains a copolymer having a (meth)acrylic acid and dialkyl diallyl ammonium salt as structural units and a fatty acid polyglycerin ester, and suppresses hair from spreading under high humidity.

(Patent Document 1) JP-A-2005-187400
(Patent Document 2) JP-A-2008-308415

[Summary of Invention]

The present invention provides a hair treatment agent comprising the following components (A) to (D) and water:

(A) succinic acid or a salt thereof: 0.1 mass% or more and 2 mass% or less in terms of succinic acid;
(B) erythritol: 0.5 mass% or more and 15 mass% or less;
(C) at least one aromatic alcohol selected from the group consisting of benzyl alcohol, phenoxyethanol and benzyloxyethanol; and
(D) at least one of polyoxyalkylene glycol with an alkylene group having 2 or 3 carbon atoms: 0.01 mass% or more and 5 mass% or less,
wherein the mass ratio of component (C) to component (D), (C)/(D), is 0.1 or more and 20 or less.

[Detailed Description of the Invention]

Recent researches have elucidated that if damages applied by chemical treatments such as coloring and permanent and heat treatments such as blowing and ironing are combined with daily repeated hair care behaviors such as hair washing and brushing, hair damages are accumulated, frizzy and wavy hair are produced. As gradually acknowledged, if the shape of hair fiber is changed as mentioned above, the other hair trouble such as difficulty in hair styling by blowing and ironing occur and keeping hair style under high humidity becomes difficult.

To deal with the other hair trouble as mentioned above, Patent Document 1 discloses that a hair cosmetic described in the Document suppresses peeling of cuticle caused by friction during hair washing. However, this Document is silent about suppressing a shape change of hair fiber, improvement of shaping in styling and keeping hair style under high humidity. While, Patent Document 2 discloses that the hair cosmetic disclosed therein can suppress spreading under high humidity of hair repeatedly damaged by a chemical treatment, a heat treatment and hair washing; however, this Document does not disclose that the hair cosmetic suppresses a shape change of hair fiber caused by damages.

Accordingly, the present invention relates to a hair cosmetic which can suppress a shape change of hair fiber caused by a combination of damages applied by chemical treatments or heat treatments with daily repeated hair care behaviors such as hair washing and brushing, thereby suppressing frizzy and wavy hair. As a result, hair strands treated with the hair treatment agent of the present invention are uniformly directed and are easily styled by blowing and ironing, and hair style can be maintained under high humidity.

The present inventors found that a shape change of hair fiber, which is caused by a combination of damages applied by chemical treatments or heat treatments with daily repeated hair care behaviors such as hair washing and brushing, can be suppressed by adding succinic acid, erythritol, particular aromatic alcohol and polyoxyalkylene glycol

to a hair treatment agent such as a shampoo, a conditioner and a hair treatment, in order to prevent frizzy and wavy hair being occurred. As a result, such a hair treatment agent enables hair strands to are uniformly directed and hair to be easily styled, and further prevents occurrence of cowlick under high humidity and produces an excellent effect of keeping hair style including manageability.

[Component (A): succinic acid or a salt thereof]

In the present invention, succinic acid or a salt thereof denoted as component (A) is used in combination with erythritol denoted as component (B), thereby exerting an effect of suppressing a shape change of hair fiber caused by a combination of damages applied by chemical treatments or heat treatments with daily repeated hair care behaviors such as hair washing and brushing.

Examples of a salt of succinic acid include an alkali metal salt, an alkaline earth metal salt, an ammonium salt, an alkanolamine salt and a basic amino acid salt. In view of e.g., availability, easiness in blending, suppression of a base-material odor of a composition and economic efficiency, a sodium salt and a potassium salt are preferable. Examples thereof include monosodium succinate, disodium succinate, monopotassium succinate and dipotassium succinate.

These components (A) can be used alone or in combination of two or more. The amount of component (A) in the hair treatment agent is 0.1 mass% or more in terms of succinic acid (more specifically, as a succinic acid equivalent in the case of a salt thereof), preferably 0.3 mass% or more and more preferably 0.4 mass% or more, in order to suppress a shape change of hair fiber by damage. In view of handling dried hair in styling and keeping hair style for a long period of time, the amount is 2 mass% or less, preferably 1.5 mass% or less and more preferably 1 mass% or less.

[Component (B): erythritol]

In the present invention, erythritol denoted as component (B) is used in combination with succinic acid or a salt thereof denoted as component (A), thereby exerting an effect of suppressing a shape change of hair fiber caused by a combination of damages applied by chemical treatments or heat treatments with daily repeated hair care behaviors such as hair washing and brushing.

The amount of component (B) in the hair treatment agent is 0.5 mass% or more, preferably 1 mass% or more and more preferably 2.5 mass% or more; and 15 mass% or less, preferably 10 mass% or less and more preferably 7.5 mass% or less, in order to suppress a shape change of hair fiber by damage.

[Component (C): particular aromatic alcohol]

An aromatic alcohol denoted as component (C) is at least one selected from the group consisting of benzyl alcohol, phenoxyethanol and benzyloxyethanol. These aromatic alcohols are used in combination with component (D) described later, thereby exerting an effect of efficiently penetrating the component (A) and component (B) into the hair.

The amount of component (C) in the hair treatment agent is preferably 0.01 mass% or more, more preferably 0.1 mass% or more and further preferably 0.15 mass% or more; and preferably 10 mass% or less, more preferably 5 mass% or less, further preferably 4 mass% or less, further preferably 2.5 mass% or less and further preferably 1 mass%, in order to penetrate the component (A) and component (B) into the hair and stably dissolve component (C) in the hair treatment agent.

The mass ratio of component (A) to component (C), (A)/(C), is preferably 0.1 or more, more preferably 0.2 or more, further preferably 0.4 or more, further preferably 0.5 or more and further preferably 0.7 or more; and preferably 10 or less, more preferably 6 or less and further preferably 4 or less, in order to efficiently penetrate the component (A) into the hair.

[Component (D): polyoxyalkylene glycol]

At least one of polyoxyalkylene glycol having an alkylene group of 2 to 3 carbon atoms and denoted as component (D) is used in combination with the aforementioned component (C), thereby exerting an effect of efficiently penetrating the component (A) and component (B) into the hair; at the same time, an effect of suppressing hair dryness after drying.

The polyoxyalkylene glycol denoted as the component (D) is a condensation product of ethylene glycol or propylene glycol and may be any one of a condensation product of ethylene glycol, a condensation product of propylene glycol and a co-condensation product of ethylene glycol and propylene glycol. These are polyoxyalkylene glycols having an average degree of condensation of 2 or more. A polyoxyalkylene glycol having an average degree of condensation of 2 or more and 10 or less is preferable and a polyoxyalkylene glycol having an average degree of condensation of 2 or more and 7 or less is more preferable. Examples of component (D) include diethylene glycol, triethylene glycol, tetraethylene glycol, polyethylene glycol (average condensation degree: 7), polyethylene glycol (average degree of

condensation: 10), dipropylene glycol, tripropylene glycol, tetrapropylene glycol, polypropylene glycol (average degree of condensation: 7) and polypropylene glycol (average degree of condensation: 10). Of them, a polypropylene glycol having an average degree of condensation of 2 or more and 7 or less is preferable, and further dipropylene glycol and polypropylene glycol (average degree of condensation 7) are preferable.

The amount of component (D) in the hair treatment agent is 0.01 mass% or more, preferably 0.05 mass% or more and further preferably 0.1 mass% or more; and 5 mass% or less, preferably 3.5 mass% or less, more preferably 2 mass% or less and further preferably 1 mass% or less, in order to penetrate the component (A) and component (B) into the hair by the combination with the aforementioned component (C), and obtain moist feeling of hair after application of the hair treatment agent.

The mass ratio of component (C) to component (D), (C)/(D), is 0.1 or more, preferably 0.15 or more and more preferably 0.25 or more; and 20 or less, preferably 10 or less, more preferably 5 or less and further preferably 3 or less, in order to efficiently penetrate the component (A) and component (B) into the hair.

A shape change of hair fiber caused by a combination of damages applied by chemical treatments or heat treatments with daily repeated hair care behaviors such as hair washing and brushing can be suppressed by coexistence of components (C) and (D) in a hair treatment agent, thereby efficiently penetrating the hair with component (A) and (B). As a result, hair strands treated with the hair treatment agent of the present invention flow in the same direction and are easily styled by blowing and ironing and further suppressed in occurrence of cowlick under high humidity, and have excellent continuity of hair style such as manageability.

[Water]

The hair treatment agent of the present invention further comprises water. As the water, e.g., ion exchanged water and distilled water can be used. The amount of water in the hair treatment agent can be balance, a amount obtained by subtracting amounts of components other than water from a total amount of the hair treatment agent; is preferably 50 mass% or more, more preferably 60 mass% or more; and preferably 98 mass% or less and more preferably 95 mass% or less.

[Anionic surfactant]

To the hair treatment agent of the present invention, further an anionic surfactant can be added. Particularly, if the hair treatment agent of the present invention is a hair cleansing agent (shampoo), an anionic surfactant is preferably contained. As the anionic surfactant, sulfate, sulfonate and carboxylate surfactants are preferable. Examples thereof include an alkyl sulfate, a polyoxyalkylene alkyl ether sulfate, a polyoxyalkylene alkenyl ether sulfate, an alkyl sulfosuccinate, a sulfosuccinic acid alkylene alkyl phenyl ether sulfate, an alkane sulfonate, a higher fatty acid salt and an alkyl ether carboxylic acid or a salt thereof. Of them, a polyoxyalkylene alkyl ether sulfate and an alkyl sulfate are preferable and further a compound represented by the following formula (1) or (2) is preferable.

$$R^1O(CH_2CH_2O)_nSO_3M \quad (1)$$

$$R^1OSO_3M \quad (2)$$

where $R^1$ represents an alkyl group or an alkenyl group having 10 to 18 carbon atoms; M represents an alkali metal, an alkaline earth metal or ammonium; and n represents a numeral of 0.5 to 5 (in terms of weight average).

Of them, a polyoxyethylene alkyl ether sulfate represent by formula (1) where $R^1$ represents an alkyl group having 12 to 14 carbon atoms; n represents a numeral of 0.5 to 3 (in terms of weight average); and M represents ammonium or sodium, is preferable, in order to obtain quick foaming and satisfactory feeling of foam in balance.

The anionic surfactants can be used alone or in combination of two or more. The amount of an anionic surfactant in the hair treatment agent of the present invention, in view of foamability and washability, is preferably 1 mass% or more, more preferably 5 mass% or more, further preferably 8 mass% or more and further preferably 10 mass% or more; and in view of liquid property in use, preferably 30 mass% or less, more preferably 25 mass% or less and further preferably 20 mass% or less.

[Water-soluble cationic polymer]

To the hair treatment agent of the present invention, further a water-soluble cationic polymer can be contained. Particularly, if the hair treatment agent of the present invention is a hair cleansing agent (shampoo), a water-soluble cationic polymer is preferably contained. The water-soluble cationic polymer improves the deposition rate of component (A) to hair surface through coacervation by a combination with the aforementioned anionic surfactant, with the result

that occurrence of cowlick under high humidity can be further suppressed.

Examples of the water-soluble cationic polymer include cationic polymers such as cationized cellulose, cationic starch, cationized guar gum, diallyl quaternary ammonium salt/acrylamide copolymer, a vinyl imidazolium trichloride/vinyl pyrrolidone copolymer, a hydroxyethyl cellulose/dimethyl diallyl ammonium chloride copolymer, a vinyl pyrrolidone/quaternized dimethylaminoethyl methacrylate copolymer, a polyvinyl pyrrolidone/alkylamino acrylate copolymer, a polyvinyl pyrrolidone/alkylamino acrylate/vinyl caprolactam copolymer, a vinyl pyrrolidone/methacrylamidopropyl trimethyl ammonium chloride copolymer, an alkyl acrylamide/acrylate/alkyl aminoalkyl acrylamide/polyethylene glycol methacrylate copolymer, an adipic acid/dimethyl amino hydroxypropylethylene triamine copolymer (U.S. Sandoz Ltd., Karutarechin), described in JP-A-H11-71435, JP-A-S53-139734 and JP-A-S60-36407. A cationized cellulose derivative, a cationized guar gum derivative, a diallyl quaternary ammonium salt/acrylamide copolymer, and a N,N-dimethylaminoethylmethacrylate diethylsulfate/N,N-dimethylacrylamide/polyethylene glycol dimethacrylate copolymer are preferable.

Examples of commercial products of the water-soluble cationic polymer which may be used in the invention include Merquat 550 ((the Lubrizol Corporation, an acrylamide/diallyldimethylammonium salt copolymer; INCI Name: polyquaternium-7), Luviquat FC370 (BASF, a copolymer of 1-vinyl-2-pyrrolidone and 1-vinyl-3-methylimidazolium salt; INCI Name: polyquaternium-16), GAFQUAT 755N (Ashland Inc., a copolymer of 1-vinyl-2-pyrrolidone and dimethylaminoethyl methacrylate; INCI Name: polyquaternium-11), Ucare polymer JR and Ucare LR series (Dow Chemical, a salt of a reaction product between trimethylammonium substituted epoxide and hydroxyethyl cellulose; INCI Name: polyquaternium-10), Poise C-60H, Poise C-80M, Poise C-150L (Kao Corp. a salt of a reaction product between trimethylammonium substituted epoxide and hydroxyethyl cellulose; INCI Name: polyquaternium-10), SOFCARE KG-301W, SOFCARE KG-101W-E (Kao Corp., N,N-dimethylaminoethyl methacrylic acid diethyl sulfate/N,N-dimethyl acrylamide/polyethylene glycol dimethacrylate copolymer; INCI Name: polyquaternium-52) and Jaguar series (Solvay, guar hydroxypropyl triammonium chloride).

The water-soluble cationic polymers can be used alone or in combination of two or more. The amount of a water-soluble cationic polymer in the hair treatment agent of the present invention is preferably 0.01 mass% or more, more preferably 0.05 mass% or more, further preferably 0.1 mass% or more, further preferably 0.2 mass% or more, further preferably 0.3 mass% or more and further preferably 0.4 mass% or more, in order to suppress occurrence of cowlick under high humidity; and is preferably 5 mass% or less, further preferably 2 mass% or less and further preferably 1 mass% or less, in order not to inhibit foamability of the hair treatment agent.

[Cationic surfactant]

To the hair treatment agent of the present invention, further, a cationic surfactant can be contained. Particularly, if the hair treatment agent of the present invention is a hair conditioner or a hair treatment, a cationic surfactant is preferably contained. As the cationic surfactant, a tertiary amine derivative or a salt thereof and a quaternary ammonium salt are mentioned.

As the tertiary amine derivative or a salt thereof, a compound selected from the group consisting of (i) an ether amine or a salt thereof and (ii) an amidoamine or a salt thereof is mentioned.

The ether amine (i) is a compound represented by the following formula (3):

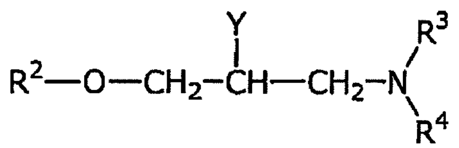
(3)

where $R^2$ represents a linear or branched alkyl group or alkenyl group having 6 to 24 carbon atoms; $R^3$ and $R^4$, which may be the same or different, each represent an alkyl group having 1 to 6 carbon atoms or $-(A^1O)_mH$ ($A^1$ represents an alkylene group having 2 to 4 carbon atoms, m represents a numeral from 1 to 6, $(A^1O)_m$ may be the same or different and the sequence thereof are arbitrarily set); and Y represents a hydrogen atom or a hydroxyl group.

Preferable examples of the ether amine or a salt thereof include N,N-dimethyl-3-hexadecyloxypropylamine or a salt thereof, N,N-dimethyl-3-octadecyloxypropylamine or a salt thereof, hexadecyloxy(2-hydroxypropyl)dimethylamine or a salt thereof and octadecyloxy(2-hydroxypropyl)dimethylamine or a salt thereof. N,N-dimethyl-3-octadecyloxypropylamine or a salt thereof and octadecyloxy(2-hydroxypropyl)dimethylamine or a salt thereof are more preferable.

The amidoamine (ii) is a compound represented by the following formula (4).

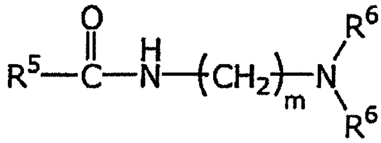 (4)

where $R^5$ represents a linear or branched alkyl group having 17 to 21 carbon atoms, two $R^6$ represent the same alkyl groups having 1 to 4 carbon atoms and m represents a numeral from 2 to 4.

Preferable examples of the amidoamine or a salt thereof include stearic acid dimethylaminoethylamide or a salt thereof, stearic acid dimethylaminopropylamide or a salt thereof, stearic acid diethylaminoethylamide or a salt thereof, stearic acid diethylaminopropylamide or a salt thereof, stearic acid dipropylaminoethylamide or a salt thereof, stearic acid dipropylaminopropylamide or a salt thereof, behenic acid dimethylaminopropylamide or a salt thereof and behenic acid diethylaminopropylamide or a salt thereof. Stearic acid dimethylaminopropylamide or a salt thereof, stearic acid diethylaminoethylamide or a salt thereof, behenic acid dimethylaminopropylamide or a salt thereof and behenic acid diethylaminopropylamide or a salt thereof are more preferable.

As the quaternary ammonium salt, more specifically, compounds represented by the following formula (5) are mentioned.

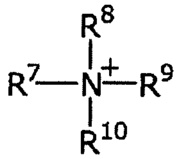 $Z^-$ (5)

where at least one of $R^7$, $R^8$, $R^9$ and $R^{10}$ represents an alkyl group or an alkenyl group, which is optionally substituted by an alkoxy group, an alkenyloxy group, an alkanoylamino group or an alkenoylamino group and has 8 to 28 carbon atoms in total; the remaining ones each independently represent a benzyl group or an alkyl group or a hydroxyalkyl group having 1 to 5 carbon atoms; and $Z^-$ represents an anion.

Of $R^7$, $R^8$, $R^9$ and $R^{10}$, $R^7$ is preferably an unsubstituted alkyl group or an alkyl group substituted with an alkoxy group, having 12 to 24 and further preferably 16 to 22 carbon atoms in total. It is preferable that $R^8$, $R^9$ and $R^{10}$, each independently represent an alkyl group having 1 to 5 carbon atoms in total. Examples of anion $Z^-$ include halide ions such as chloride ion, bromide ion; and inorganic/organic anions such as a methosulfate ion, an ethosulfate ion, a methophosphate ion, an ethophosphate ion and a methocarbonate ion. A halide ion is preferable and a chloride ion is further preferable.

Preferable examples of the quaternary ammonium salts represented by formula (5) include a cetyl trimethyl ammonium salt, a stearyl trimethyl ammonium salt, a behenyl trimethyl ammonium salt, a cetyloxypropyl trimethyl ammonium salt and a stearoxypropyl trimethyl ammonium salt.

The cationic surfactants can be used alone or in combination of two or more. The amount of a cationic surfactant in the hair treatment agent of the present invention is preferably 0.005 mass% or more, more preferably 0.01 mass% or more, further preferably 0.015 mass% or more and further preferably 0.5 mass% or more; and preferably 5 mass% or less, more preferably 4 mass% or less and further preferably 3.5 mass% or less.

[Saturated aliphatic alcohol]

To the hair treatment agent of the present invention, further a saturated fatty alcohol can be contained. Particularly, if the hair treatment agent of the present invention is a hair conditioner or a hair treatment, a saturated aliphatic alcohol is preferably contained. As the saturated aliphatic alcohol, saturated aliphatic alcohols having 12 to 28 carbon atoms are mentioned. Of these, a saturated aliphatic alcohol having 12 to 22 carbon atoms is preferable in order to improve uniformity of adhesion to hair and stability of the hair treatment agent by forming gel with a cationic surfactant. Examples of the alcohol include lauryl alcohol, myristyl alcohol, cetyl alcohol, stearyl alcohol and behenyl alcohol.

The saturated aliphatic alcohols can be used alone or in combination of two or more. The amount of a saturated aliphatic alcohol in the hair treatment agent of the present invention is preferably 0.1 mass% or more, further preferably 0.5 mass% or more, further preferably 1.0 mass% or more, further preferably 2.0 mass% or more, further preferably 3.0 mass% or more and further preferably 4.0 mass% or more; and preferably 20 mass% or less, further preferably 15 mass% or less and further preferably 10 mass% or less.

[pH]

In order to highly penetrate hair with succinic acid denoted as component (A), the pH of a 20-fold (by mass) diluted solution of the hair treatment agent of the present invention with water at 25°C is preferably 8 or less, more preferably 7 or less, further preferably 6.5 or less, further preferably 6.3 or less, further preferably 6 or less and further preferably 5.5 or less; and preferably 2 or more, more preferably 2.3 or more and further preferably 2.5 or more.

[Product form]

As the product form of the hair treatment agent according to the present invention, not only a shampoo (hair cleansing agent) but also an agent other than a hair cleansing agent such as a hair conditioner, a hair treatment, a hair styling agent, a hair lotion, a hair gel and a pre-shampoo agent, are mentioned.

[Other optional components]

To the hair treatment agent of the present invention, further, other components commonly used in hair cosmetics can be blended according to the purpose. Examples thereof include, an amphoteric surfactant such as alkyldimethylaminoacetic acid betaine, fatty acid amidopropyl betaine and alkylhydroxysulfobetaine; an ester such as isopropyl palmitate, isopropyl myristate, octyldodecyl myristate, hexyl laurate, cetyl lactate, propylene glycol monostearate, oleyl oleate, hexadecyl 2-ethyl hexanoate, isononyl isononanoate and tridecyl isononanoate; a wax such as bees wax, spermaceti, lanolin and carnauba wax; an alcohol such as ethanol, 1-propanol, 2-propanol, butanol, ethylene glycol, propylene glycol, methyl carbitol, ethyl carbitol, propyl carbitol, butyl carbitol, triethylene glycol monoethyl ether, triethylene glycol monobutyl ether and glycerin; a silicone such as dimethylpolysiloxane and amino-modified silicone; an anti-dandruff agent such as zinc pyrithione and benzalkonium chloride; a vitamin agent; a . bactericidal agent; an anti-inflammatory agent; a preservative; a chelating agent; a moisturizing agent such as panthenol; a coloring agent such as a dye and a pigment; an extract such as eucalyptus extract with a polar solvent, a protein obtained from seashell having a pearl layer or a pearl or a hydrolysate, a protein obtained from silk or a hydrolysate thereof, a protein-containing extract obtained from seeds of a leguminous plant, a ginseng extract, a rice germ extract, a fucus extract, a camellia extract, an aloe extract, an alpinia speciosa leaf extract and a chlorella extract; a pearl powder such as titanium mica; a fragrance; an ultraviolet absorber; an antioxidant; and other components described in ENCYCLOPEDIA OF SHAMPOO INGREDIENTS (MICELLE PRESS).

The hair treatment agent of the present invention may be applied to hair and allowed to dry; alternatively, applied to hair and massaged well and then washed away; and further preferably applied to hair and massaged well and then washed away.

Preferable aspects of the present invention in connection with the aforementioned embodiments are further disclosed below.

<1> A hair treatment agent comprising the following components (A) to (D) and water:

(A) succinic acid or a salt thereof: 0.1 mass% or more and 2 mass% or less in terms of succinic acid;
(B) erythritol: 0.5 mass% or more and 15 mass% or less;
(C) at least one aromatic alcohol selected from the group consisting of benzyl alcohol, phenoxyethanol and benzyloxyethanol; and
(D) at least one of polyoxyalkylene glycol with an alkylene group having 2 or 3 carbon atoms: 0.01 mass% or more and 5 mass% or less,
wherein the mass ratio of component (C) to component (D), (C)/(D), is 0.1 or more and 20 or less.

<2> The hair treatment agent according to <1>, in which the amount of component (A) in the hair treatment agent in terms of succinic acid is preferably 0.3 mass% or more and more preferably 0.4 mass% or more; and preferably 1.5 mass% or less and more preferably 1 mass% or less.

<3> The hair treatment agent according to <1> or <2>, in which the amount of component (B) in the hair treatment agent is preferably 1 mass% or more and more preferably 2.5 mass% or more; and 15 mass% or less, preferably 10 mass% or less and more preferably 7.5 mass% or less.

<4> The hair treatment agent according to any one of <1> to <3>, in which the amount of component (C) in the hair treatment agent is preferably 0.01 mass% or more, more preferably 0.1 mass% or more and further preferably 0.15 mass% or more; and preferably 10 mass% or less, more preferably 5 mass% or less, further preferably 4 mass% or less, further preferably 2.5 mass% or less and further preferably 1 mass%.

<5> The hair treatment agent according to any one of <1> to <4>, in which the mass ratio of component (A) to

component (C), (A)/(C), is preferably 0.1 or more, more preferably 0.2 or more, further preferably 0.4 or more, further preferably 0.5 or more and further preferably 0.7 or more; and preferably 10 or less, more preferably 6 or less and further preferably 4 or less.

<6> The hair treatment agent according to any one of <1> to <5>, in which the component (D) is preferably a polyoxyalkylene glycol having an average degree of condensation of 2 or more, more preferably a polyoxyalkylene glycol having an average degree of condensation of 2 or more and 10 or less, further preferably a polyoxyalkylene glycol having an average degree of condensation of 2 or more and 7 or less, further preferably a polypropylene glycol having an average degree of condensation of 2 or more and 7 or less and further preferably a dipropylene glycol or polypropylene glycol (average degree of condensation: 7).

<7> The hair treatment agent according to any one of <1> to <6>, in which the amount of component (D) in the hair treatment agent is preferably 0.05 mass% or more and further preferably 0.1 mass% or more; and preferably 3.5 mass% or less, more preferably 2 mass% or less and further preferably 1 mass% or less.

<8> The hair treatment agent according to any one of <1> to <7>, in which the mass ratio of component (C) to component (D), (C)/(D), is preferably 0.15 or more and more preferably 0.25 or more; and preferably 10 or less, more preferably 5 or less and further preferably 3 or less.

<9> The hair treatment agent according to any one of <1> to <8>, in which the amount of water in the hair treatment agent is preferably 50 mass% or more and more preferably 60 mass% or more; and preferably 98 mass% or less and more preferably 95 mass% or less.

<10> The hair treatment agent according to any one of <1> to <9>, being a hair cleansing agent.

<11> The hair treatment agent according to <10>, preferably further comprising an anionic surfactant and a water-soluble cationic polymer.

<12> The hair treatment agent according to <11>, in which the anionic surfactant is preferably selected from the group consisting of an alkyl sulfate, a polyoxyalkylene alkyl ether sulfate, a polyoxyalkylene alkenyl ether sulfate, an alkyl sulfosuccinate, a sulfosuccinic acid alkylene alkyl phenyl ether sulfate, an alkane sulfonate, a higher fatty acid salt and an alkyl ether carboxylic acid or a salt thereof.

<13> The hair treatment agent according to <11> or <12>, in which the amount of the anionic surfactant in the hair treatment agent is preferably 1 mass% or more, more preferably 5 mass% or more, further preferably 8 mass% or more and further preferably 10 mass% or more; and preferably 30 mass% or less, more preferably 25 mass% or less and further preferably 20 mass% or less.

<14> The hair treatment agent according to any one of <11> to <13>, in which the water-soluble cationic polymer is selected from the group consisting of a cationized cellulose derivative, a cationized guar gum derivative, a diallyl quaternary ammonium salt/acrylamide copolymer and a N,N-dimethylaminoethyl methacrylic acid diethyl sulfate/N,N-dimethyl acrylamide-polyethylene glycol dimethacrylate copolymer.

<15> The hair treatment agent according to <11> or <14>, in which the amount of the water-soluble cationic polymer in the hair treatment agent is preferably 0.01 mass% or more, more preferably 0.05 mass% or more, further preferably 0.1 mass% or more, further preferably 0.2 mass% or more, further preferably 0.3 mass% or more and further preferably 0.4 mass% or more; and preferably 5 mass% or less, further preferably 2 mass% or less and further preferably 1 mass% or less.

<16> The hair treatment agent according to any one of <1> to <9>, being an agent other than a hair cleansing agent.

<17> The hair treatment agent according to <16>, preferably further comprising a cationic surfactant and a saturated aliphatic alcohol.

<18> The hair treatment agent according to <17>, in which the cationic surfactant is a compound selected from the group consisting of an ether amine or a salt thereof, an amidoamine or a salt thereof and a quaternary ammonium salt.

<19> The hair treatment agent according to <17> or <18>, in which the amount of the cationic surfactant in the hair treatment agent is preferably 0.005 mass% or more, more preferably 0.01 mass% or more, further preferably 0.015 mass% or more and further preferably 0.5 mass% or more; and preferably 5 mass% or less, more preferably 4 mass% or less and further preferably 3.5 mass% or less.

<20> The hair treatment agent according to any one of <17> to <19>, in which the saturated aliphatic alcohol is a saturated aliphatic alcohol having carbon atoms of preferably 12 to 28 and more preferably 12 to 22.

<21> The hair treatment agent according to any one of <17> to <20>, in which the saturated aliphatic alcohol is selected from the group consisting of lauryl alcohol, myristyl alcohol, cetyl alcohol, stearyl alcohol and behenyl alcohol.

<22> The hair treatment agent according to any one of <17> to <21>, in which the amount of saturated aliphatic alcohol in the hair treatment agent is preferably 0.1 mass% or more, further preferably 0.5 mass% or more, further preferably 1.0 mass% or more, further preferably 2.0 mass% or more, further preferably 3.0 mass% or more and further preferably 4.0 mass% or more; and preferably 20 mass% or less, further preferably 15 mass% or less and further preferably 10 mass% or less.

<23> The hair treatment agent according to any one of <1> to <22>, in which pH of a 20-fold (by mass) diluted solution of the hair treatment agent with water at 25°C is preferably 8 or less, more preferably 7 or less, further

preferably 6.5 or less, further preferably 6.3 or less, further preferably 6 or less and further preferably 5.5 or less; and preferably 2 or more, more preferably 2.3 or more and further preferably 2.5 or more.
<24> A method for treating hair, comprising applying the hair treatment agent according to any one of <1> to <23> to hair and massaging the hair treatment agent into the hair, followed by washing.
<25> A method for treating hair, comprising applying the hair treatment agent according to any one of <1> to <23> to hair and allowing the hair treatment agent to stand still for drying.

[Examples]

Example 1, Comparative Examples 1 to 2

In accordance with the formulations shown in Table 1, individual components were stirred at room temperature to prepare hair treatment agents as homogeneous solutions. With respect to these hair treatment agents, a shape change suppression rate was evaluated according to the following Evaluation method 1.

(Evaluation method 1)

Hair tress (length 5 cm) each constituted of 8 untreated Japanese hair strands (straight or wavy hair strands having a curl radius of 10 to 20 mm) were prepared. The hair tress were treated with a bleach (Agent I and Agent II of "Prettia" foam bleach manufactured by Kao Corp. were mixed in accordance with the product instruction, allowed to stand still at room temperature for 30 minutes, infinite bath) four times.

Each of the hair tress was washed twice with the following shampoo for evaluation. Subsequently, 8 hair strands of the tress were collected up and a weight (0.3 g) was added to the tip thereof, and the tress was allowed to dry naturally for 5 minutes. Then, the weight was removed from the hair tress and the curl radius of each strand was measured. An average curl radius of 8 hair strands was specified as "curl radius (mm) before treatment".

The hair tress prepared in this manner were separately soaked in infinite baths of hair treatment agents (Example 1, Comparative Examples 1 and 2) shown in Table 1 and allowed to stand still at 40°C for 3 hours. Thereafter, the hair tress were rinsed with tap water of 40°C for 15 seconds and dried with towel. Subsequently, 8 hair strands of each tress were collected up and a weight (0.3 g) was added to the tip thereof and the tress was allowed to dry naturally for 5 minutes. Then, the weight was removed from the hair tress and the curl radius of each strand was measured again. An average curl radius of 8 hair strands was specified as "curl radius (mm) after treatment".

The curl radius used herein is determined as follows. Various circles different in radius by 1 mm were concentrically drawn. Then, a hair strand was placed along the arcs. The radius of the arc having the closest curve to the hair strand was employed as the curl radius.

Based on the curl radius values of hair before and after the treatment, the curl-radius change rate of hair was calculated in accordance with the following Equation 1. The shape-change suppression rates of individual hair treatment agents were calculated using the obtained curl-radius change rate of hair based on the hair curl-radius change rate of Comparative Example 2 (water) in accordance with Equation 2. The result's are shown in Table 1.

(Equation 1)

Hair curl-radius change rate

= curl radius (mm) after treatment/curl radius (mm) before treatment

(Equation 2)

Shape change suppression rate of hair treatment agent

= (hair curl-radius change rate of hair treatment agent/hair curl-radius change rate of Comparative Example 2) × 100

Formulation of Shampoo for evaluation

| | Amounts of component (mass%) |
|---|---|
| Sodium laureth sulfate | 15.5 |
| Lauramide DEA | 1.5 |
| Sodium benzoate | 0.5 |
| EDTA-2Na | 0.3 |
| Phosphoric acid | pH adjusted to 7 |
| Ion exchanged water | balance |

[Table 1]

| (mass %) | | Example | Comparative Example | |
|---|---|---|---|---|
| | | 1 | 1 | 2 |
| (A) | Succinic acid | 1 | - | - |
| (A') | Malic acid | - | 1 | - |
| (B) | Erythritol | 5 | 5 | - |
| (C) | Benzylalcohol | 1 | 1 | - |
| (D) | Dipropylene glycol | 0.5 | 0.5 | - |
| | Water | 92.5 | 92.5 | 100 |
| Total | | 100 | 100 | 100 |
| Mass ratio (C)/(D) | | 2 | 2 | - |
| Mass ratio (A)/(C) | | 1 | 0 | - |
| pH (of 20-fold dilution by mass) | | 3.34 | 3.00 | 7.37 |
| Shape change suppression rate of hair treatment agent (%) | | 133 | 109 | 100 |

Examples 2 to 11, Comparative Examples 3 to 10

In accordance with the formulations shown in Table 2 and Table 3, individual components were stirred at room temperature to prepare hair treatment agents as homogeneous solutions. With respect to these hair treatment agents, a shape change suppression rate was evaluated according to the following Evaluation method 2.

(Evaluation method 2)

Hair tress (length 10 cm) each constituted of 10 strands of untreated Japanese hair (straight or wavy hair strands having a curl radius of 10 to 20 mm) were prepared. The hair tress were treated with a bleach (Agent I and Agent II of "Prettia" foam bleach manufactured by Kao Corp. were mixed in accordance with the product instruction, allowed to stand still at room temperature for 30 minutes, infinite bath) four times.

Each of the hair tress was washed twice with the same shampoo for evaluation used in Evaluation method 1. Subsequently, 10 hair strands of the tress were collected up and a weight (0.3 g) was added to the tip thereof and the tress was allowed to dry naturally for 5 minutes. Then, the weight was removed from the hair tress and the curl radius of each strand was measured. Curl radius of each hair strand was measured at a distance of about 3 cm from the tip and specified as "curl radius (mm) before treatment".

Then, to simulate hair cuticle peeling due to friction applied by e.g., brushing, a hair surface was roughened. To describe it more specifically, the root of the hair tress as mentioned above was immobilized to a table with tape. A single hair strand was selected and the tip of the strand was grasped with tweezers and immobilized. The hair strand was slightly pressed down with a piece of sandpaper (manufacturer name: Nihon Kenshi Co., Ltd., waterproof abrasive paper, item number: RRCC-S, count (granularity): 400) in the form of a strip (a width of 5 mm and a length of 2 cm) and

rubbed three times in the direction from the tip to the root over 3 cm or more. The hair tress thus prepared were separately soaked in infinite baths of hair treatment agents (Example 2 to 11, Comparative Examples 3 to 10) shown in Table 2 or Table 3 and allowed to stand still at 40°C for 3 hours. Thereafter, the hair tress were rinsed with tap water of 30°C for 15 seconds and dried with towel. Subsequently, 10 hair strands per tress were collected up and a weight (0.3 g) was added to the tip thereof and the tress was allowed to dry naturally for 15 minutes. Then, the weight was removed from the hair tress and the curl radius of each strand was measured at a distance of about 3 cm from the tip as is the same as mentioned above and specified as "curl radius (mm) after treatment". In measurement, 6 to 10 intact hair strands were selected from 10 hair strands. The curl radius used herein is determined as follows. Various circles different in radius by 1 mm were concentrically drawn. Then a hair strand was placed along the arcs. The radius of the arc having the closest curve to the hair strand was employed as the curl radius.

Based on the same hair before and after the treatment, the curl-radius change rate of the hair was calculated in accordance with the following Equation 3. Further, the hair strands treated in the same manner was measured in the same manner and an average value was obtained and used as an average curl-radius change rate. Subsequently, using the average curl-radius change rate of the hair thus obtained, the shape change suppression rates of individual hair treatment agents were calculated in accordance with Equation 4, based on the hair average curl-radius change rate of Comparative Example 3 (water). The results are shown in Table 2 and Table 3.

(Equation 3)

Hair curl-radius change rate

= curl radius (mm) after treatment/curl radius (mm) before treatment

(Equation 4)

Shape change suppression rate of hair treatment agent

= (average hair curl-radius change rate of hair treatment agent/average hair curl-radius change rate of Comparative Example 3) × 100

[Table 2]

| (mass %) | | Example | | | | | | | | | |
|---|---|---|---|---|---|---|---|---|---|---|---|
| | | 2 | 3 | 4 | 5 | 6 | 7 | 8 | 9 | 10 | 11 |
| (A) | Succinic acid | 1 | 1 | 0.4 | 1 | 2 | 1 | 1 | 1 | 1 | 1 |
| (B) | Erythritol | 5 | 5 | 5 | 5 | 5 | 0.5 | 15 | 5 | 5 | 5 |
| (C) | Benzylalcohol | 1 | 4 | 4 | 0.1 | 1 | 1 | 1 | 0.15 | 1 | 1 |
| (D) | Dipropylene glycol | 0.5 | 5 | 5 | 0.5 | 0.5 | 0.5 | 0.5 | 0.5 | 0.05 | 0.05 |
| | Water | 92.5 | 85 | 85.6 | 93.4 | 91.5 | 97 | 82.5 | 93.35 | 92.95 | 92.95 |
| Total | | 100 | 100 | 100 | 100 | 100 | 100 | 100 | 100 | 100 | 100 |
| Mass ratio (C)/(D) | | 2 | 0.8 | 0.8 | 0.2 | 2 | 2 | 2 | 0.3 | 20 | 20 |
| Mass ratio (A)/(C) | | 1 | 0.25 | 0.1 | 10 | 2 | 1 | 1 | 6.67 | 1 | 1 |
| pH (of 20-fold dilution by mass) | | 3.34 | 3.33 | 3.42 | 3.33 | 3.18 | 3.34 | 3.34 | 3.33 | 3.33 | 3.33 |

(continued)

| (mass %) | Example | | | | | | | | | |
|---|---|---|---|---|---|---|---|---|---|---|
| | 2 | 3 | 4 | 5 | 6 | 7 | 8 | 9 | 10 | 11 |
| Shape change suppression rate of hair treatment agent (%) | 144 | 130 | 121 | 141 | 118 | 130 | 159 | 147 | 157 | 162 |

[Table 3]

| (mass %) | | Example | | Comparative Example | | | | | | | |
|---|---|---|---|---|---|---|---|---|---|---|---|
| | | 2 | 3 | 3 | 4 | 5 | 6 | 7 | 8 | 9 | 10 |
| (A) | Succinic acid | 1 | 1 | - | - | 1 | 1 | 1 | 1 | 1 | 1 |
| (B) | Erythritol | 5 | 5 | - | 5 | - | 5 | 5 | 5 | 5 | 5 |
| (C) | Benzylalcohol | 1 | 4 | - | 4 | 4 | - | 4 | - | 1 | 5 |
| (D) | Dipropylene glycol | 0.5 | 5 | - | 5 | 5 | 5 | - | - | 9 | 9 |
| | Water | 92.5 | 85 | 100 | 86 | 90 | 89 | 90 | 94 | 84 | 80 |
| Total | | 100 | 100 | 100 | 100 | 100 | 100 | 100 | 100 | 100 | 100 |
| Mass ratio (C)/(D) | | 2 | 0.8 | - | 0.8 | 0.8 | 0 | - | - | 0.11 | 0.56 |
| Mass ratio (A)/(C) | | 1 | 0.25 | - | 0 | 0.25 | - | 0.25 | - | 1 | 0.2 |
| pH (of 20-fold dilution by mass) | | 3.34 | 3.33 | 7.37 | 5.63 | 3.33 | 3.35 | * | 3.36 | 3.34 | 3.33 |
| Shape change suppression rate of hair treatment agent (%) | | 144 | 130 | 100 | 102 | 96 | 98 | * | 103 | 106 | 107 |
| *) Since a solution was separated, either pH measurement or evaluation was not carried out. | | | | | | | | | | | |

Example 12, Comparative Examples 11 and 12

In accordance with the formulations shown in Table 4, individual components were mixed while stirring at 80°C and cooled to normal temperature to prepare shampoos. With respect to these shampoos, an effect of suppressing spread of a hair tress was evaluated, in accordance with the following method.

(Preparation of hair tress for evaluation)

Using Japanese hair, hair tress having a length of 25 cm and a weight of 2 g were prepared. The hair tress were treated with a bleach (Agent I and Agent II of "Prettia" foam bleach manufactured by Kao Corp. were mixed in accordance with the product instruction, allowed to stand still at room temperature for 30 minutes, infinite bath) twice. The hair tress were washed twice with the same shampoo for evaluation, rinsed with tap water of 40°C for 15 seconds and dried with towel. Thereafter, the hair tress was dried by a hot-air dryer. This process (washing the hair tress with the shampoo for evaluation, rinsing with tap water at 40°C for 15 seconds, drying with towel, and further drying the hair bundle by the hot-air dryer) was repeated 60 times to obtain hair tress for evaluation.

(Suppression of spread of hair tress)

The hair tress for evaluation was moistened with water. Shampoos (2 g) shown in Table 4 were separately applied to the tress and foamed for 30 seconds. Subsequently, the hair tress were rinsed with tap water of 40°C for 30 seconds, dried with towel and then dried with cool air. This operation was repeated 5 times provided that natural drying was employed instead of cold-air drying in the fifth process (operation). It took about 30 minutes for the hair tress to completely dry. Then, the width of each of the hair tress was measured by the method described later.
Thereafter, the root of each of the hair tress was sandwiched by the plates of iron (Straight Iron ES2400,

manufactured by Recca Electronics) previously set at a temperature of 180°C and then the plates were slid from the root toward the tip of the hair tress at a rate of 2.5 cm/sec while sandwiching the hair tress to straighten the hair. This operation was repeated three times. The hair tress of this state was hanged at 25°C, in an environment with a humidity of 90% RH and allowed to stand still for 20 hours. Then, the width of the hair tress was measured in accordance with the method shown below.

The width (d) of a hair tress at a distance of 20 cm from the root was measured at the time after natural drying (X) before ironing, after ironing (Y) and after placing in an environment of high humidity for 20 hours (Z). The width values of the hair tress of individual cases were represented by dX, dY and dZ and the degree of spread was obtained based on the following Equation 5. The results are shown in Table 4.

(Equation 5)

$$\text{Degree of spread} = [(dZ-dY)/(dX-dY)] \times 100$$

[Table 4]

| (mass%: all: amount of active ingredient) | | | | Example | Comparative Example | |
|---|---|---|---|---|---|---|
| | Ingredient name | Manufacturer | INCI name | 12 | 11 | 12 |
| (A) | | | Succinic acid | 0.7 | - | 0.7 |
| (A') | | | Malic acid | - | 02 | - |
| (A') | | | Citric acid | - | 0.17 | - |
| (B) | | | Erythritol | 5 | - | - |
| (C) | | | Benzyl alcohol | 0.3 | 0.3 | 0.3 |
| (C) | | | Phenoxyethanol | 0.4 | 0.4 | 0.4 |
| (D) | ADEKA CARPOL DL-30 | ADEKA | PPG-7 | 0.4 | 0.4 | 0.4 |
| | EMAL 125A | KAO Corp. | Ammonium Laureth sulfate | 12.5 | 12.5 | 12.5 |
| | AKYPO LM-26C | KAO Corp. | Laureth-4 acetate | 1.45 | 1.45 | 1.45 |
| | AMPHITOL 20HD | KAO Corp. | Lauryl hydroxysultaine | 1.2 | 1.2 | 1.2 |
| | EMULGEN 116 | KAO Corp. | Laureth-16 | 0.5 | 0.5 | 0.5 |
| | KALCOL 4098 | KAO Corp. | Myristyl alcohol | 0.4 | 0.4 | 0.4 |
| | PENETOL GE-ID | KAO Corp. | Isodecyl glyceryl ether | 0.468 | 0.468 | 0.468 |
| | KAOSOFCARE GP-1 | KAO Corp. | PPG-3 Caprylyl ether | 0.55 | 0.55 | 0.55 |
| | Crodacid 18-MEA | Croda Europe | Lanolin fatty acid | 0.075 | 0.075 | 0.075 |
| | FARMIN DM E-80 | KAO Corp. | Stearoxypropyl dimethyl amine | 0.054 | 0.054 | 0.054 |
| | Jaguar C-14SK (US)(CN) | Solvay | Guar hydroxypropyltrimonium chloride | 0.15 | 0.15 | 0.15 |
| | CATICELO L-150 | KAO Corp. | Polyquaternium-10 | 0.18 | 0.18 | 0.18 |

(continued)

| (mass%: all: amount of active ingredient) | | | | Example | Comparative Example | |
|---|---|---|---|---|---|---|
| | Ingredient name | Manufacturer | INCI name | 12 | 11 | 12 |
| | SOFCARE KG-101 W-E | KAO Corp. | Polyquaternium-52 | 0.10368 | 0.10368 | 0.10368 |
| | EMANON PCSA-M2 | KAO Corp. | Glycol distearate | 1.36 | 1.36 | 1.36 |
| | Silicone KHE-1 | Shin-Etsu Chemical Co., Ltd. | Dimethicone | 1.2 | 1.2 | 12 |
| | Hybrid sunflower seed oil | YOKOZEKI OIL & FAT INDUSTRIES CO., LTD. | Sunflower seed oil | 0.04 | 0.04 | 0.04 |
| | | | Modified alcohol | 0.33 | 0.33 | 0.33 |
| | | | Sodium hydroxide | 0.2 | 0.2 | 0.2 |
| | | | Fragrance | 0.55 | 0.55 | 0.55 |
| | | | Purified water | Balance | Balance | Balance |
| Mass ratio (C)/(D) | | | | 1.75 | 1.75 | 1.75 |
| Mass ratio (A)/(C) | | | | 1 | 0 | 1 |
| pH (of 20-fold dilution by mass) | | | | 5.9 | 5.9 | 5.9 |
| Degree of spread | | | | 31.15 | 85.19 | 41.25 |

It was found that when the shampoo of the present invention is used, a shape change of hair fiber due to damage is suppressed, spread of a hair tress is suppressed, in other words, a hair style can be kept more than before.

Example 13: Shampoo

| | |
|---|---|
| (1) Succinic acid | 0.5 mass% |
| (2) Erythritol | 2.5 mass% |
| (3) Benzyl alcohol | 0.5 mass% |
| (4) Phenoxyethanol | 0.2 mass% |
| (5) Polypropylene glycol-7 | 0.2 mass% |
| (6) Laureth sulfate ammonium | 11.5 mass% |
| (7) Lauryl hydroxysultaine | 1.65 mass% |
| (8) Guar hydroxypropyltrimonium chloride | 0.14 mass% |
| (9) Polyquaternium-10 | 0.14 mass% |
| (10) Polyquaternium-52 | 0.1 mass% |
| (11) Dimethicone | 0.3 mass% |
| (12) Sodium hydroxide | 0.4 mass% |
| (13) Fragrance | 0.6 mass% |
| (14) Water | Balance |

When the shampoo having the above formulation is used, a shape change due to hair damage is suppressed, spread of hair tress is suppressed, in other words, hair style is excellently kept for a long period of time.

Example 14: Hair conditioner

| | |
|---|---|
| (1) Succinic acid | 0.5 mass% |
| (2) Erythritol | 4 mass% |
| (3) Benzyl alcohol | 0.15 mass% |
| (4) Dipropylene glycol | 0.55 mass% |
| (5) Behentrimonium chloride | 1.03 mass% |
| (6) Steartrimonium chloride | 0.25 mass% |
| (7) Cetanol | 2.1 mass% |
| (8) Stearyl alcohol | 2.35 mass% |
| (9) Dimethicone | 1.5 mass% |
| (10) Fragrance | 0.5 mass% |
| (11) water | Balance |

When the conditioner having the above formulation is used, a shape change of hair fiber due to damage is suppressed, spread of a hair tress is suppressed, in other words, hair style is excellently kept for a long period of time.

## Claims

**1.** A hair treatment agent comprising the following components (A) to (D) and water:

(A) succinic acid or a salt thereof: 0.1 mass% or more and 2 mass% or less in terms of succinic acid;
(B) erythritol: 0.5 mass% or more and 15 mass% or less;
(C) at least one aromatic alcohol selected from the group consisting of benzyl alcohol, phenoxyethanol and benzyloxyethanol; and
(D) at least one of polyoxyalkylene glycol with an alkylene group having 2 or 3 carbon atoms: 0.01 mass% or more and 5 mass% or less,
wherein a mass ratio of component (C) to component (D), (C)/(D), is 0.1 or more and 20 or less.

**2.** The hair treatment agent according to Claim 1, wherein a mass ratio of component (A) to component (C), (A)/(C), is 0.1 or more and 10 or less.

**3.** The hair treatment agent according to Claim 1 or 2, being a hair cleansing agent.

**4.** The hair treatment agent according to Claim 3, further comprising an anionic surfactant and a water-soluble cationic polymer.

**5.** The hair treatment agent according to Claim 1 or 2, being an agent other than a hair cleansing agent.

**6.** The hair treatment agent according to Claim 5, further comprising a cationic surfactant and a saturated aliphatic alcohol.

**7.** A method for treating hair, comprising applying the hair treatment agent according to any one of Claims 1 to 6 to the hair and massaging the hair treatment agent into the hair, followed by washing.

## Patentansprüche

**1.** Haarbehandlungsmittel, das die folgenden Komponenten (A) bis (D) und Wasser umfasst:

(A) Bernsteinsäure oder ein Salz davon: 0,1 Massen-% oder mehr und 2 Massen-% oder weniger in Bezug auf Bernsteinsäure;
(B) Erythrit: 0,5 Massen-% oder mehr und 15 Massen-% oder weniger;
(C) mindestens einen aromatischen Alkohol ausgewählt aus der Gruppe bestehend aus Benzylalkohol, Phenoxyethanol und Benzyloxyethanol; und
(D) mindestens ein Polyoxyalkylenglykol mit einer Alkylengruppe mit 2 oder 3 Kohlenstoffatomen: 0,01 Massen-% oder mehr und 5 Massen-% oder weniger,
wobei ein Massenverhältnis der Komponente (C) zur Komponente (D), (C)/(D), 0,1 oder mehr und 20 oder weniger beträgt.

2. Haarbehandlungsmittel gemäß Anspruch 1, wobei ein Massenverhältnis der Komponente (A) zur Komponente (C), (A)/(C), 0,1 oder mehr und 10 oder weniger beträgt.

3. Haarbehandlungsmittel gemäß Anspruch 1 oder 2, welches ein Haarreinigungsmittel ist.

4. Haarbehandlungsmittel gemäß Anspruch 3, das ferner ein anionisches Tensid und ein wasserlösliches kationisches Polymer umfasst.

5. Haarbehandlungsmittel gemäß Anspruch 1 oder 2, welches ein anderes Mittel als ein Haarreinigungsmittel ist.

6. Haarbehandlungsmittel gemäß Anspruch 5, das ferner ein kationisches Tensid und einen gesättigten aliphatischen Alkohol umfasst.

7. Verfahren zur Behandlung von Haar, welches es umfasst, dass das Haarbehandlungsmittel gemäß mindestens einem der Ansprüche 1 bis 6 auf das Haar aufgetragen wird und das Haarbehandlungsmittel in das Haar einmassiert wird gefolgt von Waschen.

**Revendications**

1. Agent de traitement capillaire comprenant les composants (A) à (D) suivants et de l'eau :

(A) acide succinique ou un sel de celui-ci : 0,1 % en masse ou plus et 2 % en masse ou moins en termes d'acide succinique ;
(B) erythritol : 0,5 % en masse ou plus et 15 % en masse ou moins ;
(C) au moins un alcool aromatique sélectionné à partir du groupe constitué par de l'alcool benzylique, du phénoxyéthanol et du benzyloxyéthanol ; et
(D) au moins un polyoxyalkylène glycol avec un groupe alkylène ayant 2 ou 3 atomes de carbone : 0,01 % en masse ou plus et 5 % en masse ou moins,
dans lequel le rapport en masse du composant (C) au composant (D), (C) / (D) est égal à 0,1 ou plus et à 20 ou moins.

2. Agent de traitement capillaire selon la revendication 1, dans lequel un rapport en masse du composant (A) au composant (C), (A) / (C) est égal ou supérieur à 0,1 et égal ou inférieur à 10.

3. Agent de traitement capillaire selon la revendication 1 ou 2, étant un agent de nettoyage des cheveux.

4. Agent de traitement capillaire selon la revendication 3, comprenant en outre un tensioactif anionique et un polymère cationique hydrosoluble.

5. Agent de traitement capillaire selon la revendication 1 ou 2, étant un agent autre qu'un agent de nettoyage des cheveux.

6. Agent de traitement capillaire selon la revendication 5, comprenant en outre un tensioactif cationique et un alcool aliphatique saturé.

7. Procédé de traitement capillaire, comprenant l'application de l'agent de traitement capillaire selon l'une quelconque des revendications 1 à 6 sur les cheveux et le massage de l'agent de traitement capillaire dans les cheveux, suivi d'un lavage.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005187400 A
- JP 2008308415 A
- JP H1171435 A
- JP S53139734 A
- JP S6036407 A


### Non-patent literature cited in the description


- ENCYCLOPEDIA OF SHAMPOO INGREDIENTS. MICELLE PRESS